# EUROPÄISCHE PATENTANMELDUNG

(11) **EP 0 761 667 A1**
(43) Veröffentlichungstag der Anmeldung: **12.03.1997**
(21) Anmeldenummer: 96113160.4
(22) Anmeldetag: 16.08.1996
(51) Int. Cl.: C07D 405/12, A61K 31/40, C07D 409/12

(54) **Hetercyclisch substituierte Pseudopeptide**

(30) Priorität: 29.08.1995 DE 19531685
(71) Anmelder: BAYER AG, 51368 Leverkusen (DE)
(72) Erfinder: Raddatz, Siegfried, Dr., 51065 Köln (DE); Wild, Hanno, Dr., Orange, Connecticut 06477 (US); Hansen, Jutta, Dr., 42115 Wuppertal (DE)

(57) **Zusammenfassung**

Die vorliegende Erfindung betrifft neue heterocyclisch substituierte Pseudopeptide der allgemeinen Formel (I) 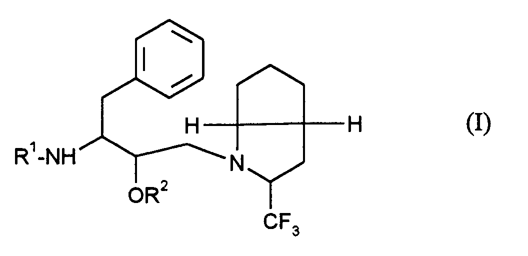 in welcher
die Substituenten die in der Beschreibung angegebene Bedeutung haben, ein Verfahren zu ihrer Herstellung und ihre Verwendung als anti-retrovirale Mittel.

## Beschreibung

Die vorliegende Erfindung betrifft neue heterocyclisch substituierte Pseudopeptide, ein Verfahren zu ihrer Herstellung und ihre Verwendung als anti-retrovirale Mittel.

Trifluoromethyl-haltige Pseudopeptide sind in der Publikation EP 528 242 als anti-retrovirale Mittel beschrieben.

Die vorliegende Erfindung betrifft neue heterocyclisch substituierte Pseudopeptide der allgemeinen Formel (I) 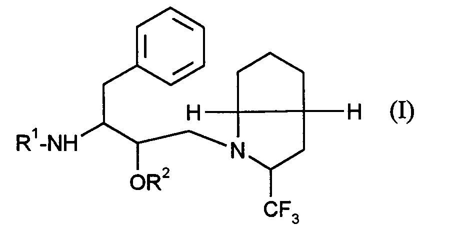 in welcher
- R¹: für einen Rest der Formel 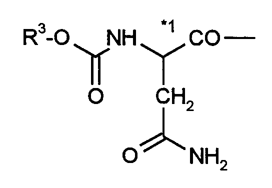 oder R⁴―O―CO-steht,
worin
R³ und R⁴ gleich oder verschieden sind und einen heterocyclischen Rest der Formel 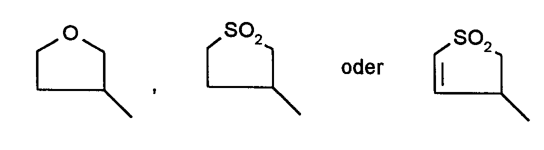 bedeuten,
- R²: für Wasserstoff oder für geradkettiges oder verzweigtes Acyl mit bis zu 20 Kohlenstoffatomen steht, das gegebenenfalls durch Carboxy, durch geradkettiges oder verzweigtes Alkoxycarbonyl mit bis zu 6 Kohlenstoffatomen substituiert ist,
und deren Salze.

Die erfindungsgemäßen Verbindungen der allgemeinen Formel (I) haben mehrere asymmetrische Kohlenstoffatome.

Der repräsentative Rest der allgemeinen Formel (Ia) 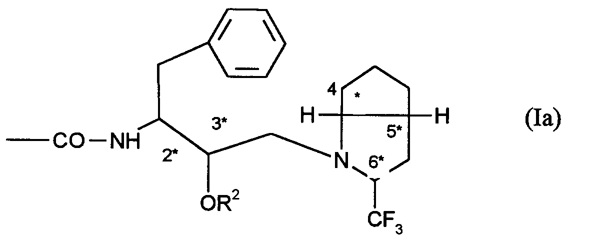 besitzt 5 asymmetrische Kohlenstoffatome (*), die unabhängig voneinander in der. R- oder S-Konfiguration vorliegen. Bevorzugt sind die Konfigurationen 1(R), 2(R), 3(S), 4(S), 5(S) und 6(S).

Die in den erfindungsgemäßen Verbindungen der allgemeinen Formel (I) eingesetzten Aminosäuren können sowohl in der L- als auch in der D-Form vorliegen.

Physiologisch unbedenkliche Salze der heterocyclisch substituierten Pseudopeptide mit trifluoromethyl-substituiertem 2-Azabicyclooctan können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Bevorzugt sind die erfindungsgemäßen Verbindungen der allgemeinen Formel (I), in welcher
- R¹: für einen Rest der Formel 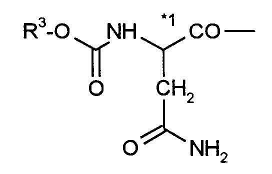 oder R⁴―O―CO-steht,
worin
R³ und R⁴ gleich oder verschieden sind und einen heterocyclischen Rest der Formel 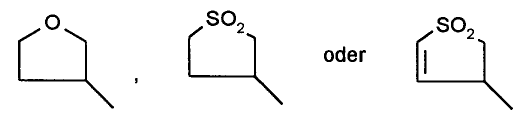 bedeutet,
- R²: für Wasserstoff oder für geradkettiges oder verzweigtes Acyl mit bis zu 6 Kohlenstoffatomen steht,
und deren Salze.

Besonders bevorzugt sind die erfindungsgemäßen Verbindungen der allgemeinen Formel (I),
in welcher
- R¹: für einen Rest der Formel 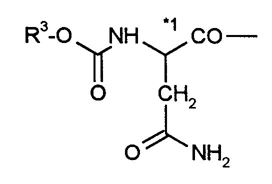 oder R⁴―O―CO-steht,
worin
R³ und R⁴ gleich oder verschieden sind und einen heterocyclischen Rest der Formel 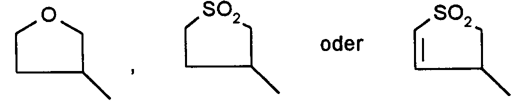 bedeuten,
- R²: für Wasserstoff oder für geradkettiges oder verzweigtes Acyl mit bis zu 5 Kohlenstoffatomen steht,
und deren Salze.

Ganz besonders bevorzugt sind die erfindungsgemäßen Verbindungen der allgemeinen Formel (I),
in welcher
- R²: für Wasserstoff oder für geradkettiges oder verzweigtes Acyl mit bis zu 4 Kohlenstoffatomen steht.

Außerdem wurde ein Verfahren zur Herstellung der erfindungsgemäßen Verbindungen der allgemeinen Formel (I) gefunden, dadurch gekennzeichnet, daß man

Verbindungen der Formel (II) oder (III) 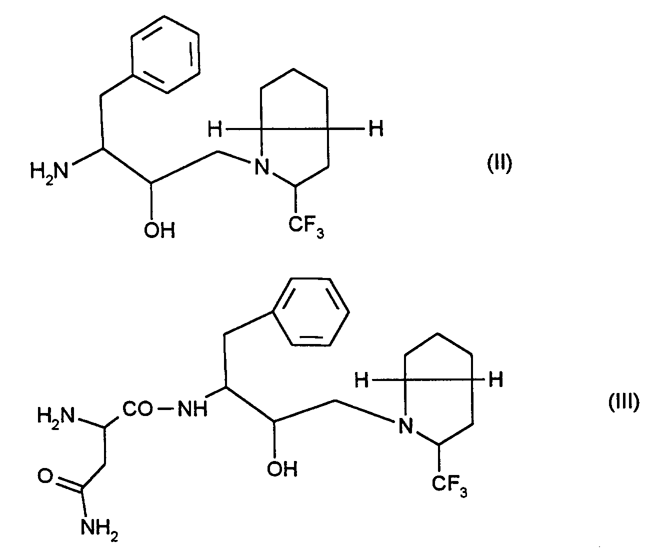 mit Verbindungen der allgemeinen Formel (IV),

R¹-OH (IV)

in welcher
- R¹: die oben angegebene Bedeutung hat,
gegebenenfalls unter vorgeschalteter Aktivierung der Carbonsäure, in inerten Lösemitteln und in Anwesenheit einer Base und/oder eines Hilfsmittels, umsetzt, und in einem zweiten Schritt im Fall R² ≠ H eine Acylierung in inerten Lösemitteln gegebenenfalls in Anwesenheit einer Base und/oder eines Hilfsmittels, durchführt.

Das erfindungsgemäße Verfahren kann durch folgendes Formelschema beispielhaft erläutert werden:

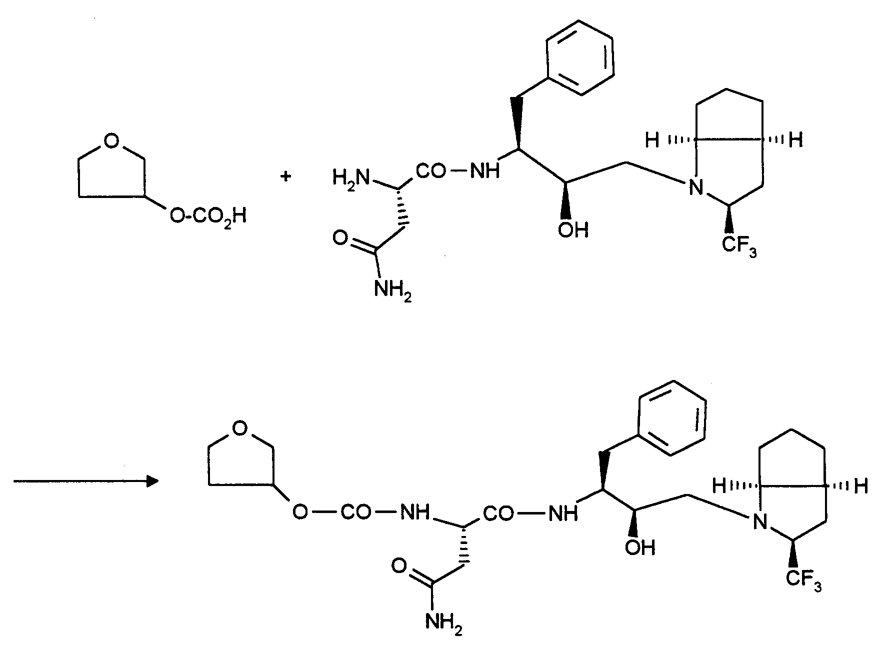

Als Lösemittel eignen sich für alle Verfahrensschritte die üblichen inerten Lösemittel, die sich unter den Reaktionsbedindungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dimethylformamid oder Tetrahydrofuran.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine, Dimethylaminopyridin oder N-Methylpiperidin, oder Amide wie Natriumamid oder Lithiumdiisopropylamid, oder Lithium-N-silylalkylamide, wie beispielsweise Lithium-N-(bis)triphenysilylamid, oder Lithiumalkyle wie n-Butyllithium.

Die Base wird in einer Menge von 1 Mol bis 10 Mol, bevorzugt von 1 Mol bis 3 Mol, bezogen auf 1 Mol der Verbindungen der Formel (II) oder (III) eingesetzt.

Als Hilfsmittel eignen sich bevorzugt Kondensationsmittel, die auch Basen sein können, insbesondere wenn die Carboxylgruppe als Anhydrid aktiviert vorliegt. Bevorzugt werden hier die üblichen Kondensationsmittel wie Carbodiimide, z.B. N,N'-Diethyl-, N,N'-Diisopropyl-, N,N'-Dicyclohexylcarbodiimid, N-(3-Dimethylaminoisopropyl)-N'-ethyl-carbodiimid-Hydrochlorid, N-Cyclohexyl-N'-(2-morpholinoethyl)-carbodiimid-metho-p-toluolsulfonat (CMCT bzw. Morpho-CDI), oder Carbonylverbindungen wie Carbonyldiimidazol, oder 1,2-Oxazoliumverbindungen wie 2-Ethyl-5-phenyl-1,2-oxazolium-3-sulfat oder 2-tert.Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tri(dimethylamino)phosphonium-hexafluorophosphat, 1-Hydroxybenzotriazol oder Dimethylaminopyridin.

Die Reaktionen können sowohl bei Normaldruck als auch bei erhöhtem oder erniedrigtem Druck (beispielsweise 0,5 bis 5 bar), bevorzugt bei Normaldruck, durchgeführt werden.

Die Umsetzung erfolgt im allgemeinen in einem Temperaturbereich von -20°C bis +40°C, bevorzugt von 0°C bis Raumtemperatur.

Die Acylierung erfolgt im allgemeinen in einem der oben aufgeführten Ether oder Halogenkohlenwasserstoffen, vorzugsweise Tetrahydrofuran oder Methylenchlorid, in einem Temperaturbereich von -30°C bis +50°C, bevorzugt von -10°C bis Raumtemperatur.

Als Basen und/oder Hilfsstoffe für die Acylierung eignen sich im allgemeinen die oben aufgeführten organischen Amine und Pyridine. Bevorzugt sind Triethylamin und Dimethylaminopyridin.

Die Base wird in einer Menge von 1 Mol bis 10 Mol, bevorzugt von 1 Mol bis 3 Mol, bezogen auf 1 Mol des jeweiligen Alkohols, eingesetzt.

Die Verbindung der allgemeinen Formel (II) ist bekannt.

Die Verbindung der Formel (III) ist neu und kann beispielsweise hergestellt werden, indem man

Verbindungen der allgemeinen Formel (V) 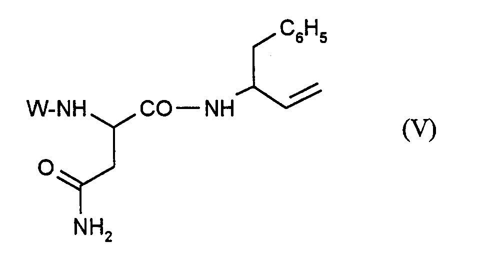 in welcher
- W: für eine Aminoschutzgruppe, vorzugsweise tert. Butoxycarbonyl, steht,
zunächst mit einer Epoxidierungsreaktion, gegebenenfalls mit Hilfe einer Base oder einer Phasentransferkatalysator, in die Verbindungen der allgemeinen Formel (VI), 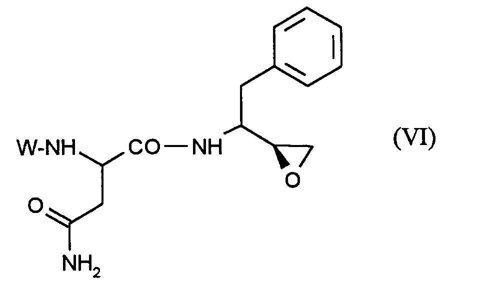 in welcher
- W: die oben angegebene Bedeutung hat,
überführt und anschließend mit 3-Trifluormethyl-2-azabicyclo[3.3.0]octan der Formel (VII) 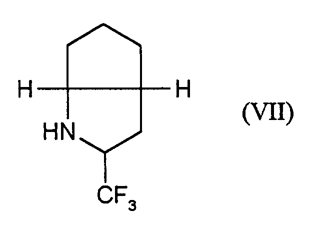 in Lösemitteln umsetzt und die Schutzgruppe nach üblichen Methoden abspaltet.

Als Lösemittel eignen sich die üblichen organischen Lösemittel, die sich unter den Reaktionsbedingungen nicht verändern. Hierzu gehören bevorzugt organische Lösemittel wie Alkohole, z.B. Methanol, Ethanol oder n-Propanol, Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Dichlorethan (DCE), Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dichlorethan, Dimethylformamid oder n-Propanol.

Als Reagentien für die Epoxidierung eignen sich die literaturbekannten Verbindungen wie beispielsweise m-Chlorperbenzoesäure, Magnesiummonoperoxyphthalat, Dimethyldioxiran oder Methyl(trifluormethyl)dioxiran. Bevorzugt sind m-Chlorperbenzoesäure und Magnesiummonoperoxyphthalat [vgl. P. Brongham et al., Synthesis (1987), 1015; W. Adam et al., J. Org. Chem. 52, 2800 (1987) und R. Curci et al., J. Org. Chem. 53, 3890 (1988)].

Wird die Epoxidierung mit Hilfe einer Phasentransfer-Katalysator durchgeführt, so werden als Hilfsstoffe beispielsweise organische Ammoniumchloride oder -bromide wie beispielsweise Benzyltriethylammoniumchlorid oder -bromid, Methyltrioctylammoniumchlorid, Tetrabutylammoniumbromid, Tricaprylmethylammoniumchlorid (Aliquat 336) eingesetzt. Bevorzugt sind Benzyltriethylammoniumchlorid und -bromid.

Die Epoxidierung wird in einem Temperaturbereich von -10°C bis +90°C, bevorzugt von 0°C bis +60°C, durchgeführt.

Die Reaktionen können sowohl bei Normaldruck als auch bei erhöhtem oder erniedrigtem Druck (beispielsweise 0,5 bis 5 bar), bevorzugt bei Normaldruck, durchgeführt werden.

Die Verbindungen der allgemeinen Formel (IV) sind an sich bekannt oder nach üblichen Methoden herstellbar.

Die Verbindungen der allgemeinen Formeln (V) und (VI) sind größtenteils bekannt [vgl. EP 528 242].

Die Verbindung der allgemeinen Formel (VII) ist neu und kann beispielsweise durch Fluorierung von 2-Azabicyclo[3.3.0]octan-3-carbonsäure in der jeweiligen Konfiguration mit HF und SF₄ hergestellt werden.

Es wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in der folgenden Literaturangabe [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen IC₅₀-Werte beziehen sich auf die Substanzkonzentration, die unter den oben aufgeführten Testbedingungen eine 50%ige Hemmung der Protease-Aktivität bewirkt.

### Enzym assay, HIV-1

**Tabelle I**

| **Beispiel-Nr.** | **HIV-1-Protease-Inhibition** | |
|---|---|---|
| | **IC**_{**50**} **[Mol/l]** | **IC**_{**95**} **[Mol/l]** |
| 1 | 3,0 x 10⁻⁸ | 3,8 x 10⁻⁷ |
| 2 | 2,7 x 10⁻¹⁰ | 3,6 x 10⁻⁹ |
| 3 | 9,5 x 10⁻⁹ | n.t. |
| 4 | 2,1 x 10⁻⁹ | 3,9 x 10⁻⁹ |
| 5 | 4,1 x 10⁻⁹ | 4,7 x 10⁻⁸ |
| 6 | 2,5 x 10⁻⁸ | 5,0 x 10⁻⁸ |
| 7 | 2,5 x 10⁻⁸ | n.t. |
| 8 | 3,2 x 10⁻⁹ | 4,1 x 10⁻⁸ |

Außerdem zeigten die erfindungsgemäßen Verbindungen Wirkung in Lentivirus infizierten Zellkulturen. Dies konnte am Beispiel des HIV-Virus gezeigt werden.

### HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phytohaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten 2¹⁰fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 Syncytien, während die unbehandelte Zellkontrolle keine Syncytien aufwies.

Die IC₅₀-Werte wurden als die Konzentration der behandelten und infizierten Zellen ermittelt, bei der 50% (ca. 10 Syncytien) der virusinduzierten Syncytien durch die Behandlung mit der erfindungsgemäßen Verbindung unterdrückt waren.

Es wurde nun gefunden, daß die erfindungsgemäßen Verbindungen HIV infizierte Zellen vor der virusinduzierten Zellzerstörung schützen.

### Zellkulturassay, PBL

**Tabelle II**

| **Beispiel-Nr.** | **PBL IC**_{**50**} **[Mol/l]** | **IC**_{**95**} **[Mol/l]** |
|---|---|---|
| 1 | 2,2 x 10⁻⁶ | 3,5 x 10⁻⁶ |
| 2 | 2,0 x 10⁻⁶ | 3,5 x 10⁻⁶ |
| 3 | 1,2 x 10⁻⁶ | 1,9 x 10⁻⁶ |
| 4 | >1,6 x 10⁻⁵ | n.t. |
| 5 | 1,4 x 10⁻⁵ | 2,2 x 10⁻⁵ |
| 6 | 2,8 x 10⁻⁶ | 1,3 x 10⁻⁵ |
| 7 | 6,2 x 10⁻⁶ | n.t. |
| 8 | 1,7 x 10⁻⁵ | >2 x 10⁻⁵ |

Die erfindungsgemäßen Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:
1.) Die Behandlung und Prophylaxe von menschlichen Retrovirusinfektionen.
2.) Die Behandlung oder Prophylaxe von durch HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.
3.) Die Behandlung oder die Prophylaxe einer HTLV-I oder HTLV-II Infektion.
4.) Die Behandlung oder die Prophylaxe des AIDS-carrier Zustandes (AIDS-Überträger-Zustand).

Als Indikationen in der Tiermedizin können beispielsweise angeführt werden:

### Infektionen mit

a) Maedi-visna (bei Schafen und Ziegen)
b) progressivem Pneumonievirus (PPV) (bei Schafen und Ziegen)
c) caprine arthritis encephalitis Virus (bei Schafen und Ziegen)
d) Zwoegersiekte Virus (bei Schafen)
e) infektiösem Virus der Anämie (des Pferdes)
f) Infektionen, verursacht durch das Katzenleukämievirus
g) Infektionen ,verursacht durch das Virus der Katzen-Immundefizienz (FIV)
h) Infektionen, verursacht durch das Virus der Affen-Immundefizienz (SIV)

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen in einer Konzentration von etwa 0,1 bis 99,5 Gew.-%, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Die erfindungsgemäßen Verbindungen sind Enzyminhibitoren und können als solche für alle Zwecke eingesetzt werden, für die Enzyminhibitoren brauchbar sind. Beispielhaft ist hier zu nennen der Einsatz als Label für Affinitätschromatographie, die Verwendung als Hilfsmittel für die Aufklärung von Enzymstrukturen und Reaktionsmechanismen sowie der Einsatz als Reagenz für Diagnostika.

### Beispiel 1

### (2S,3S)-3-(Tetrahydrofuran-3-yl-oxycarbonyl-L-asparaginyl)amino-1-[(S,S,S)-3-trifluormethyl-2-azabicyclo[3.3.0]octan-2-yl}-2-hydroxy-4-phenylbutan

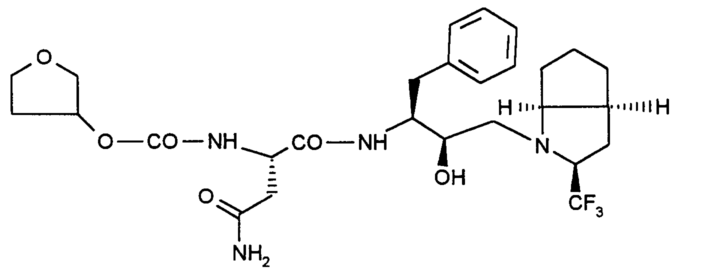

0,1 g (0,22 mmol) (2R,3S)-3-(L-Asparaginyl)amino-1-{(S,S,S)-3-trifluormethyl-2-azabicyclo[3.3.0]octan-2-yl}-2-hydroxy-4-phenylbutan und 0,11 g (0,44 mmol) Tetrahydrofuran-3-yl-4-nitrophenylcarbonat (analog Daniel F. Veber et al., J. Org. Chem., Vol. 42, No. 20, 1977, p. 3286-8) werden in 3 ml THF gelöst und unter Argonatmosphäre über Nacht bei RT gerührt. Anschließend wird zur Trockne eingedampft, und der Rückstand säulenchromatographisch getrennt (Kieselgel 60, Dichlormethan / Methanol = 100/5, R_{f} = 0,4).
Ausbeute: 120 mg (96,0% d.Th.) farbloser Schaum

In Analogie zur Vorschrift des Beispiels 1 werden die in Tabelle 1 aufgeführten Verbindungen hergestellt:

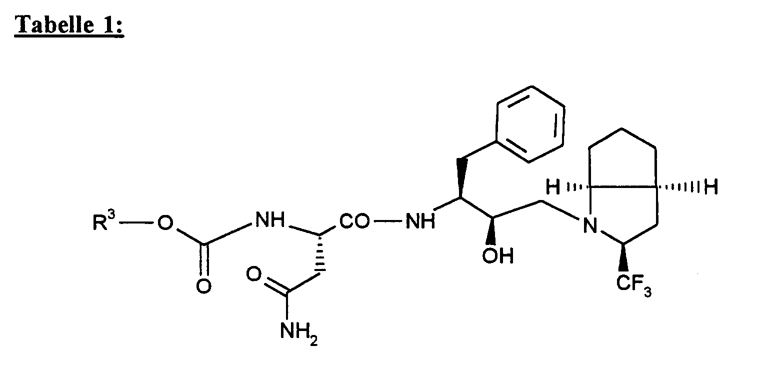

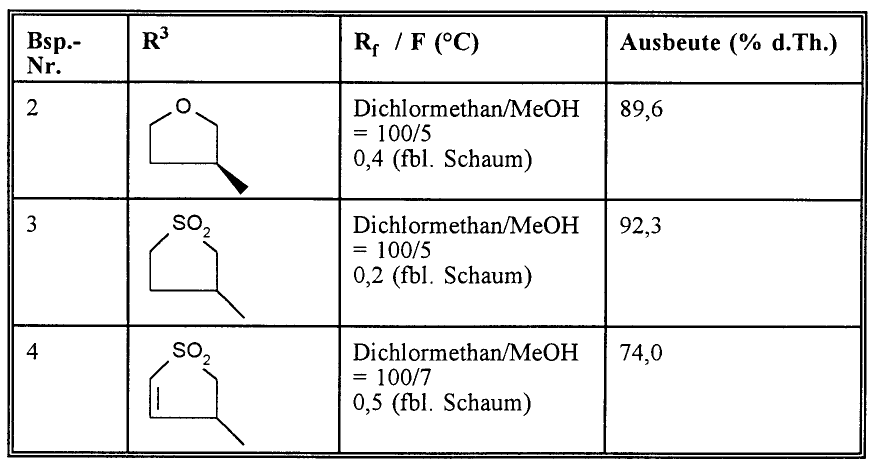

### Beispiel 5

### (2R,3S)-3-(Tetrahydrofuran-3-yl-oxycarbonyl)-amino-1-{(S,S,S)-3-trifluormethyl-2-azabicyclo[3.3.0]octan-2-yl}-2-hydroxy-4-phenylbutan

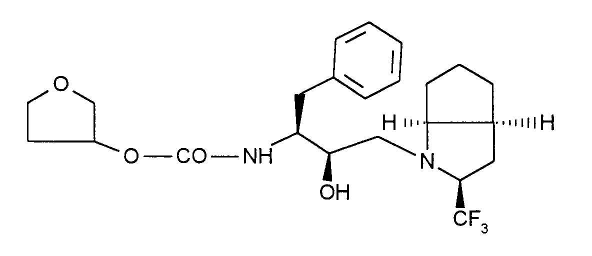

0,1 g (0,29 mmol) (2R,3S)-3-Amino-1-{(S,S,S)-3-trifluormethyl-2-azabicyclo[3.3.0]octan-2-yl}-2-hydroxy-4-phenylbutan und 0,11 g (0,44 mmol) Tetrahydrofuran-3-yl-4-nitrophenylcarbonat (analog Daniel F. Veber et al., J. Org. Chem., Vol. 42, No. 20, p. 3286 -8) werden in 3 ml abs. THF gelöst und unter Argonatmosphäre über Nacht bei RT gerührt. Anschließend wird zur Trockne eingedampft und der Rückstand säulenchromatographisch getrennt (Kieselgel 60; Dichlormethan / Essigsäureethylester = 4/1).
R_{f} = 0,5 (Dichlormethan / Essigester = 1/1)
Ausbeute: 50 mg (37,5% d.Th.) fbl. Schaum

In Analogie zur Vorschrift des Beispiels 5 werden die in Tabelle 2 aufgeführten Verbindungen hergestellt.

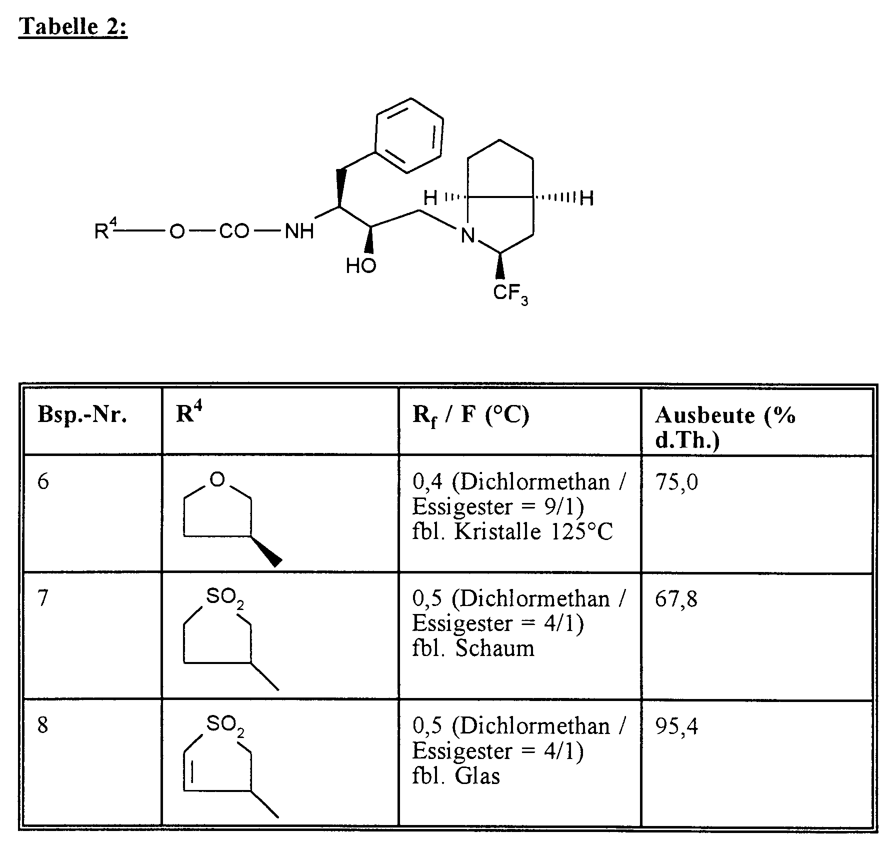

### Beispiel 9

### (2S,3S)-3-(Tetrahydrofuran-3-yl-oxycarbonyl-L-asparaginyl)amino-1-{(S,S,S)-3-trifluormethyl-2-azabicyclo[3.3.0]octane-2-yl-2-acetoxy-4-phenylbutan

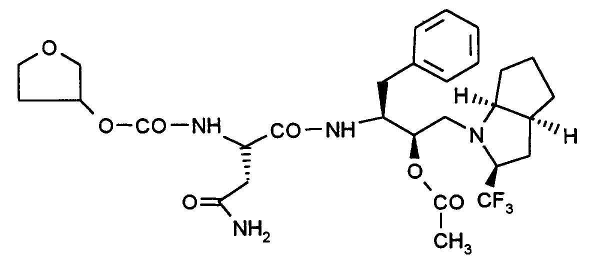

50 mg (0,0876 mmol) der Verbindung des Beispiels 1, 25 mg (0,035 ml) Triethylamin und 13 mg (0,13 mmol) Acetanhydrid werden unter Argonatmosphäre in 2 ml getrocknetem Tetrahydrofuran (THF) gelöst, mit einer Spatelspitze Dimethylaminopyridin (DMAP) versetzt und über Nacht gerührt.

Das Gemisch wird i.V. zur Trockne eingedampft und mit 5 ml Wasser und 15 ml Essigsäureethylester angerührt. Die organische Phase wird abgetrennt, mit Natriumsulfat getrocknet und säulenchromatographisch getrennt (Kieselgel 60, Laufmittel: Dichlormethan/Essigsäureethylester = 4/1).

farblose Kristalle, Fₚ = 191°C; R_{f} = 0,4 (Dichlormethan/Essigsäureethylester = 4/1), Ausbeute: 35 mg (65,2 % d.Th.).

### Beispiel 10

### (2S,3S)-3-[Tetrahydrofuran-(3S)-3-yl-oxycarbonyl]-amino-1-{(S,S,S)-3-trifluoromethyl-2-azabicyclo-[3.3.0]octan-2-yl}-2-isobutyroxy-4-phenylbutan

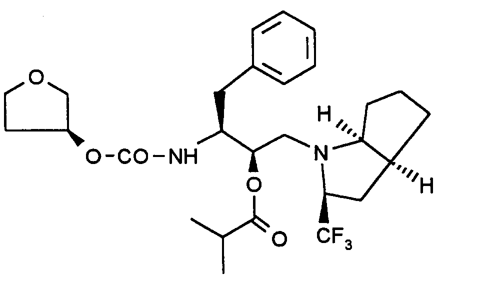

In Analogie zur Vorschrift des Beispiels 9 wird die Titelverbindung aus 50 mg (0,11 mmol) der Verbindung aus Beispiel 6, 20 mg (0,027 ml/0,2 mmol) Triethylamin und 15 mg (0,14 mmol) Buttersäureanhydrid mit einer Spatelspitze Dimethylaminopyridin hergestellt.

Farbloser Schaum; R_{f} = 0,6 (Dichlormethan/Essigsäureethylester 4:1) Ausbeute: 40 mg (69,3 % d.Th.)

## Patentansprüche

1. Heterocyclisch substituierte Pseudopeptide der allgemeinen Formel (I) 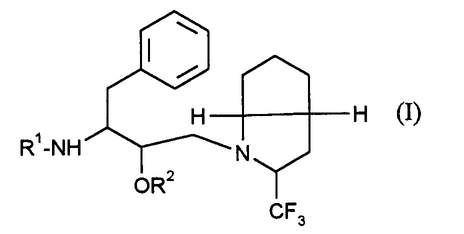 in welcher
R¹ für einen Rest der Formel 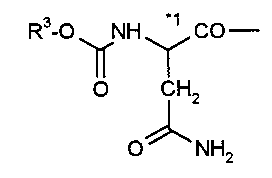 oder R⁴―O―CO-steht,
worin
R³ und R⁴ gleich oder verschieden sind und einen heterocyclischen Rest der Formel 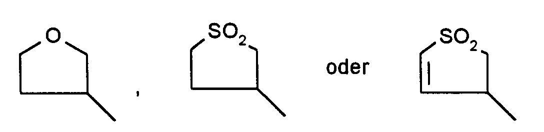 bedeuten,
R² für Wasserstoff oder für geradkettiges oder verzweigtes Acyl mit bis zu 20 Kohlenstoffatomen steht, das gegebenenfalls durch Carboxy, durch geradkettiges oder verzweigtes Alkoxycarbonyl mit bis zu 6 Kohlenstoffatomen substituiert ist,
und deren Salze.

2. Verbindungen der allgemeinen Formel (I), gemäß Anspruch 1
in welcher
R¹ für einen Rest der Formel 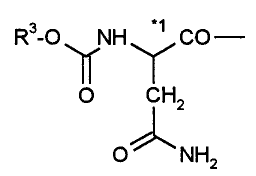 oder R⁴―O―CO-steht,
worin
R³ und R⁴ gleich oder verschieden sind und einen heterocyclischen Rest der Formel 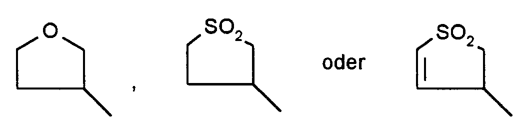 bedeutet,
R² für Wasserstoff oder für geradkettiges oder verzweigtes Acyl mit bis zu 6 Kohlenstoffatomen steht,
und deren Salze.

3. Verbindungen der allgemeinen Formel (I), gemäß Anspruch 1
in welcher
R¹ für einen Rest der Formel 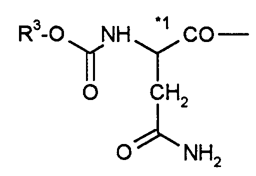 oder R⁴―O―CO-steht,
worin
R³ und R⁴ gleich oder verschieden sind und einen heterocyclischen Rest der Formel 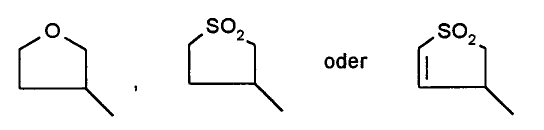 bedeutet,
R² für Wasserstoff oder für geradkettiges oder verzweigtes Acyl mit bis zu 5 Kohlenstoffatomen steht,
und deren Salze.

4. Verbindungen der allgemeinen Formel (I), gemäß Anspruch 1
in welcher
R² für Wasserstoff oder für geradkettiges oder verzweigtes Acyl mit bis zu 4 Kohlenstoffatomen steht.

5. Verfahren zur Herstellung der Verbindungen der allgemeinen Formel (I), gemäß Anspruch 1, dadurch gekennzeichnet, daß man
Verbindungen der Formel (II) oder (III) 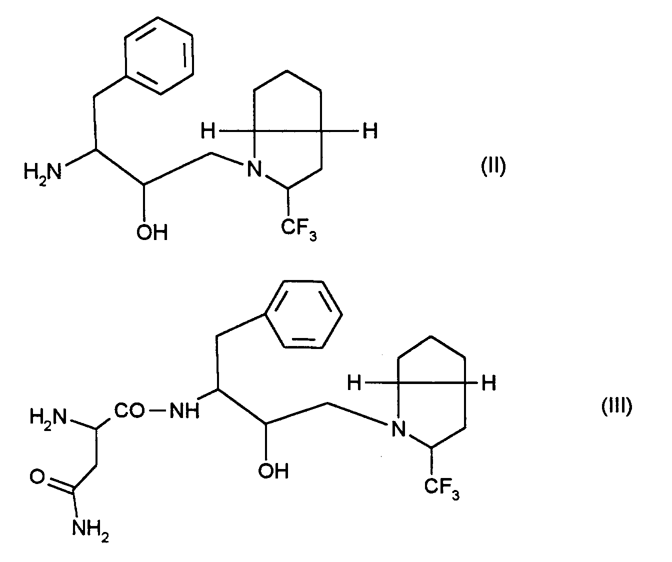 mit Verbindungen der allgemeinen Formel (IV),
R¹-OH (IV)
in welcher
R¹ die im Anspruch 1 angegebene Bedeutung hat,
gegebenenfalls unter vorgeschalteter Aktivierung der Carbonsäure, in inerten Lösemitteln und in Anwesenheit einer Base und/oder eines Hilfsmittels, umsetzt, und in einem zweiten Schritt im Fall R² ≠ H eine Acylierung in inerten Lösemitteln gegebenenfalls in Anwesenheit einer Base und/oder eines Hilfsmittels, durchführt.

6. Arzneimittel enthaltend eine oder mehrere Verbindungen aus den Ansprüchen 1 bis 4.

7. Verwendung von Verbindungen aus den Ansprüchen 1 bis 4 zur Herstellung von antiretroviralen Arzneimitteln.
